(19) Europäisches Patentamt
European Patent Office
Office européen des brevets

(11) Numéro de publication : **0 283 365 B1**

(12) # FASCICULE DE BREVET EUROPEEN

(45) Date de publication du fascicule du brevet : **10.07.91 Bulletin 91/28**

(51) Int. Cl.$^5$ : **C07K 7/06**, C07K 7/30, A61K 37/02

(21) Numéro de dépôt : **88400459.9**

(22) Date de dépôt : **29.02.88**

(54) **Nouveaux peptides dérivés de la CCK8, leur préparation et les compositions pharmaceutiques qui les contiennent.**

(30) Priorité : **02.03.87 FR 8702770**

(43) Date de publication de la demande : **21.09.88 Bulletin 88/38**

(45) Mention de la délivrance du brevet : **10.07.91 Bulletin 91/28**

(84) Etats contractants désignés : **AT BE CH DE ES FR GB GR IT LI LU NL SE**

(56) Documents cités :
**US-A- 3 705 140**
**US-A- 4 490 364**
**JOURNAL OF MEDICINAL CHEMISTRY, vol. 30, no. 6, juin 1987, pages 962-968, American Chemical Society; B. CHARPENTIER et al.: "Investigation of peripheral cholecystokinin receptor heterogeneity by cyclic and related linear analogues of CCK26-33: Synthesis and biological properties"**

(73) Titulaire : **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur : **Charpentier, Bruno**
**Le Clos Marella 6 Allée des Troenes**
**F-94240 L'Hay Les Roses (FR)**
Inventeur : **Roques, Bernard Pierre**
**12 rue Eugène Delacroix**
**F-94410 Saint Maurice (FR)**

(74) Mandataire : **Pilard, Jacques et al**
**RHONE-POULENC SANTE, Service Brevets,**
**20 Avenue Raymond Aron**
**F-92165 Antony Cédex (FR)**

EP 0 283 365 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux peptides présentant des propriétés cholécystokininergiques spécifiques des récepteurs du système nerveux central, leur préparation et les compositions thérapeutiques qui les contiennent.

On sait qu'il existe au moins deux types de récepteurs cholécystokininergiques, de poids moléculaires différents [SAKAMOTO et al., J. Biol. Chem. 258, 12707 (1983) ; SAKAMOTO et al., Biochem. Biophys. Res. Comm., 124, 497 (1984)], capables de lier l'octapeptide sulfaté suivant (connu sous le nom de CCK8) :

Asp-Tyr($SO_3H$)-Met-Gly-Trp-Met-Asp-Phe-$NH_2$ (I)

On distingue ainsi les récepteurs du système nerveux central et les récepteurs du système nerveux périphérique. Au niveau du système nerveux central, la CCK8 joue un rôle de neurotransmetteur [GOLTERMAN et al., J. Biol. Chem. 255, 6181 (1980)]. De plus, elle est colocalisée avec la dopamine dans certains neurones de la voie mésolimbique [HÖKFELT et al., Nature, 285, 476 (1980)] ; elle en antagonise les effets dans le système mésolimbique et facilite la neurotransmission dopaminergique dans le striatum [FUXE et al., Eur. J. Pharmacol., 67, 329 (1980)]. Les récepteurs périphériques, eux, sont impliqués dans la contraction des muscles lisses au niveau intestinal [HUTCHINSON et al., Eur. J. Pharmacol., 69, 87 (1981) ; CHANG et al., Neuroscience Lett., 46, 71 (1984)] et dans la libération d'amylase pancréatique [JENSEN et al., J. Biol. Chem., 257, 5554 (1982)].

La CCK8 ainsi que d'autres produits structurellement apparentés sont connus par la demande de brevet allemand 3 138 233 pour avoir une action neuropsychotrope mais leur intérêt est atténué par le fait qu'ils ne sont pas dépourvus d'une activité périphérique.

Le but de la présente invention était donc de préparer des analogues de la CCK8 conservant une activité centrale (prévisible par la mesure de leur affinité pour les récepteurs cérébraux) et si possible dépourvus d'activité périphérique ou tout au moins ne présentant cette dernière qu'à un degré moindre (l'activité périphérique se déduisant de la mesure de l'affinité des produits vis-à-vis des sites récepteurs périphériques).

Comme on sait par expérience que la moindre modification d'un ou plusieurs acides aminés dans un peptide provoque des modifications importantes et même entraîne souvent l'inactivité totale du peptide, les modifications à apporter à la CCK8 pour atteindre le but cherché étaient loin d'être évidentes. La présente invention apporte cependant la solution au problème grâce aux premiers analogues cycliques de la CCK8 revendiqués ici.

Ainsi, la présente invention concerne de nouveaux peptides dérivés de la CCK8 de formule générale :

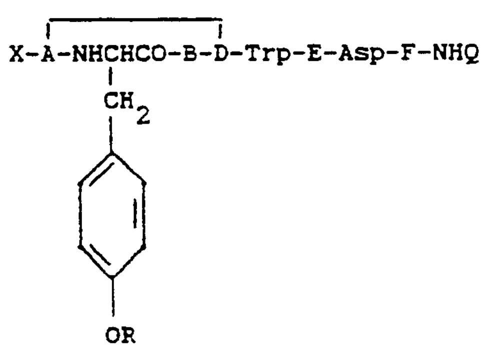

(II)

dans laquelle X représente un atome d'hydrogène ou bien un radical protecteur de la fonction amine du type acyle tel que formyle, acétyle, chloracétyle, trifluoroacétyle, propionyle, butyryle, isobutyryle, γ-chlorobutyryle, oxalyle, succinyle, glutamyle, pyroglutamyle, phtalyle, p-toluènesulfonyle, ou du type uréthane tel que tert-butyloxycarbonyle, isopropyloxycarbonyle, éthoxycarbonyle, allyloxycarbonyle, cyclohexyloxycarbonyle, benzyloxycarbonyle, p-chlorobenzyloxycarbonyle, p-bromobenzyloxycarbonyle, p-nitrobenzyloxycarbonyle,

R représente un atome d'hydrogène ou un radical $—SO_3H$,

A et D sont définis comme suit :

a) ou bien A et D sont différents et représentent un radical de formule générale :

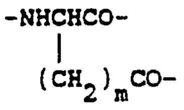 (III) ou 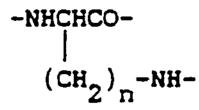 (IV)

dans lesquelles m et n représentent des nombres entiers compris entre 1 et 5, étant entendu que lorsque A représente un radical de formule générale (III) D représente un radical de formule générale (IV) et réciproquement et que A et D forment ensemble avec le reste de la molécule un cycle par amidification de leurs chaînes latérales,

b) ou bien A et D sont identiques ou différents et représentent un radical de formule générale :

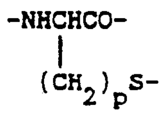 (V) ou 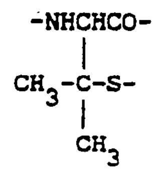 (VI)

dans lesquelles p représente un nombre entier égal à 1 ou 2, étant entendu que A et D forment ensemble avec le reste de la molécule un cycle par formation d'un pont disulfure entre les atomes de soufre de leurs chaînes latérales,

B et E, identiques ou différents, représentent un reste méthionine, nor-leucine, leucine, sérine, thréonine, allo-thréonine ou bien un reste cystéine ou homo-cystéine dont les fonctions OH ou SH peuvent être libres ou protégées

i) par un radical alcoyle contenant 1 à 6 atomes de carbone en chaîne droite ou ramifiée ou
ii) par un radical phényle non substitué ou substitué par un ou plusierus atomes de fluor ou
iii) par un radical benzyle non substitué ou substitué par un ou plusieurs atomes de fluor ou
iv) par un radical alcoylcarbonyle dont la partie alcoyle contient 1 à 5 atomes de carbone en chaîne droite ou ramifiée ou bien benzoyle, phénylacétyle ou benzhydrylcarbonyle dont les parties phényle peuvent être non substituées ou substituées par un ou plusieurs atomes de fluor,

F représente une liaison de valence ou un reste phénylalanine, tyrosine O-méthylée ou cyclohexylalanine et

Q représente un atome d'hydrogène ou un radical alcoyle contenant 1 à 6 atomes de carbone en chaîne droite ou ramifiée non substitué ou substitué par un ou plusieurs atomes de fluor, ou un radical phényle ou phénylalcoyle dont la partie alcoyle contient 1 à 3 atomes de carbone en chaîne droite ou ramifiée et dont la partie phényle peut être non substituée ou substituée par un ou plusieurs atomes de fluor, étant entendu que les différents acides aminés entrant dans la définition des produits selon l'invention se présentent sous la forme D, L ou DL, ainsi que leurs sels pharmaceutiquement acceptables.

Selon l'invention, les produits de formule générale (II) peuvent être préparés par action d'un acide de formule générale :

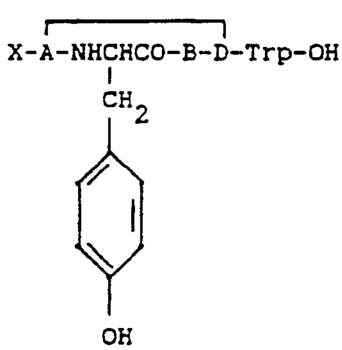

(VII)

dans laquelle les symboles sont définis comme précédemment sur une amine de formule générale :

H-E-Asp-F-NHQ (VIII)

dans laquelle les symboles sont définis comme précédemment, suivie d'une sulfatation lorsqu'on veut obtenir un produit de formule générale (II) dans laquelle R représente un radical $—SO_3H$.

Le couplage des produits de formule générale (VII) et (VIII) s'effectue par toute méthode connue de l'homme de métier pour condenser un peptide sur un autre peptide.

Il est particulièrement avantageux d'utiliser le produit de formule générale (VII) sous une forme activée. Comme forme activée, on peut citer le produit de réaction du composé de formule générale (VII) avec l'hydroxybenzotriazole, la N-hydroxysuccinimide, le p-nitrophénol ou le tri- ou penta-chlorophénol, préparé en présence d'un agent de condensation.

Dans la pratique, il est particulièrement avantageux d'utiliser, comme forme activée, le produit de réaction avec la N-hydroxysuccinimide dans un solvant organique tel qu'un éther comme le tétrahydrofuranne, un solvant chloré comme le chlorure de méthylène ou le chloroforme, un amide comme le diméthylformamide ou un mélange de ces solvants, en présence de dicyclohexylcarbodiimide à une température voisine de 0°C.

La condensation du produit de formule (VII) sous forme activée sur le produit de formule générale (VIII) s'effectue généralement dans les mêmes conditions de température et de milieu réactionnel que celles dans lesquelles a été effectuée la préparation de la forme activée du produit de formule générale (VII). Il n'est d'ailleurs pas nécessaire d'isoler cette forme activée pour effectuer la condensation sur le produit de formule générale (VIII).

La sulfatation subséquente du produit de condensation des produits de formules générales (VII) et (VIII) peut être effectuée par tout agent de sulfatation connu n'affectant pas le reste de la molécule. Il est particulièrement avantageux d'utiliser comme agent de sulfatation le complexe $SO_3$-pyridine en opérant dans un solvant organique anhydre tel que le diméthylformamide ou la pyridine ou un mélange de ces solvants à une température voisine de 20°C.

Les produits de formule générale (VII) peuvent être préparés par cyclisation des produits de formule générale :

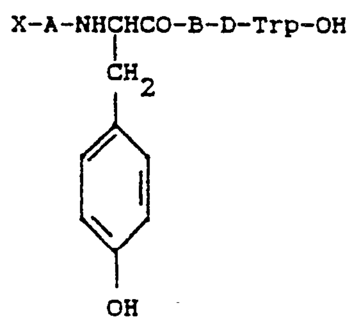

(IX)

dans laquelle les symboles sont définis comme précédemment à l'exception du fait que A et D ne sont pas cyclisés ensemble.

Lorsque A et D sont définis comme précédemment en a) la cyclisation s'effectue généralement au moyen d'un agent d'amidification tel que le diphénylphosphorylazide dans un solvant organique tel que le diméthylformamide à une température comprise entre –40°C et 4°C.

Lorsque A et D sont définis comme précédemment en b) la cyclisation s'effectue par oxydation des thiols des chaînes latérales des groupements A et D soit au moyen d'une solution de ferricyanure de potassium dans l'eau à un pH de 8,0 et à une concentration en peptide de 0,3 mM selon la technique décrite par SCHILLER et al., Biochem. Biophys. Res. Comm., 101, 337 (1981), soit en utilisant l'iode comme oxydant à une concentration en peptide de 2 mM dans le méthanol dans les conditions décrites par SCHLITTER et WEBER, Helv. Chim. Acta., 55, 2061 (1972).

Les produits de formule générale (IX) peuvent être préparés selon les méthodes habituelles connues en chimie peptidique, par exemple selon la méthode décrite précédemment pour coupler ensemble les produits de formules générales (VII) et (VIII), ou encore selon la méthode de synthèse séquentielle en phase solide, connue sous le nom de synthèse de Merrifield.

Les produits de formule générale (VIII) peuvent également être prépares selon les méthodes habituelles de la chimie peptidique.

Comme le remarquera facilement l'homme du métier, il est nécessaire pour mettre en oeuvre le procédé selon l'invention d'effectuer à divers stades de la synthèse des réactions sur des produits dont les fonctions acides, amines, alcools ou thiols auront dû au préalable être protégées. Dans ce cas, le radical protecteur sera

éliminé au moment le plus opportun de la synthèse, notamment avant couplage des acides aminés ou des peptides entre eux. A titre non limitatif, on pourra par exemple protéger les fonctions de la manière suivante :

– Pour les fonctions amines, on pourra utiliser les radicaux protecteurs énumérés pour la définition du symbole X précédent. Lorsque le blocage s'effectue sur un produit intermédiaire et est donc destiné à être éliminé par la suite, il est avantageux d'utiliser le radical tert-butyloxycarbonyle ; celui-ci pourra ensuite être éliminé dans des conditions relativement douces, par exemple en milieu acide au moyen d'acide trifluoroacétique dilué ou non, dans le chlorure de méthylène, ou au moyen d'une solution d'acide chlorhydrique gazeux dans un solvant anhydre tel que le dioxanne ou l'acide acétique ; on isole alors souvent le peptide ou l'acide aminé sous forme de trifluoroacétate ou d'acétate. La base pourra être libérée au moment que l'on désire à l'aide d'une base plus forte telle que la triéthylamine ou la N,N'-diisopropyléthylamine.
Lorsqu'en plus de la fonction amine N-terminale, il existe une autre fonction amine portée par la chaîne latérale d'un aminoacide tel que la lysine, cette fonction amine pourra être avantageusement bloquée par un autre type de radical afin de débloquer ensuite les fonctions amines sélectivement. Dans le cas de la lysine par exemple, on pourra employer un radical benzyloxycarbonyle que l'on pourra éliminer ensuite par hydrogénolyse en présence d'un catalyseur tel que le palladium sur charbon actif.

– Pour les fonctions acides, on pourra par exemple utiliser les radicaux esters habituellement employés en chimie peptidique et facilement éliminables par la suite.
Il est paticulièrement avantageux de bloquer les fonctions acides sous forme d'esters méthyliques qui seront ensuite éliminés par saponification au moyen d'une solution aqueuse d'un hydroxyde alcalin tel que la soude.
Lorsqu'en plus de la fonction acide C-terminale, il existe une autre fonction acide portée par la chaîne latérale d'un aminoacide tel que l'acide aspartique, cette fonction acide pourra être avantageusement bloquée par un autre type de radical afin de débloquer ensuite les fonctions acides sélectivement. Dans le cas de l'acide aspartique par exemple, on pourra employer un radical benzyloxy que l'on pourra éliminer ensuite par hydrogénolyse en présence d'un catalyseur tel que le palladium sur charbon actif.

– Pour les fonctions alcools et thiols, on pourra, par exemple, employer les radicaux énumérés aux points i) à iv) dans la définition des symboles B et E. Il est particulièrement avantageux d'utiliser dans le cas des fonctions thiols soit le radical nitro-2 phénylsulfényle qui pourra être éliminé par réduction au moyen du dithiothréitol dans les conditions décrites par SKALA et al., Science, 226, 444 (1984), soit le radical S-acétamidométhyle selon la technique de VEBER et coll., J. Am. Chem. Soc., 94, 5456 (1972).

Les nouveaux produits de formule générale (II) ainsi que les intermédiaires de synthèse peuvent être purifiés le cas échéant par les méthodes habituelles telles que la cristallisation, la chromatographie ou la formation de sels.

Lorsque les produits de formule générale (II) possèdent dans leur molécule une fonction amine libre, ils peuvent être convertis en sels d'addition avec les acides par action d'un acide, en opérant dans un solvant organique tel qu'un alcool, une cétone, un éther ou un solvant chloré. Le sel précipite, éventuellement après concentration de sa solution ; il est séparé par filtration ou décantation.

Comme sels d'addition avec les acides, on peut citer les sels d'acides minéraux tels que chlorhydrates, sulfates ou phosphates et les sels d'acides organiques tels qu'acétates, propionates, succinates, benzoates, fumarates, maléates, méthanesulfonates, iséthionates, théophillineacétates, salicylates, phénolphtalinates, méthylène bis-β-oxynaphtoates ou des dérivés de substitution de ces composés.

Lorsque les produits de formule générale (II) renferment dans leur molécule une ou plusieurs fonctions acides salifiables, il est généralement avantageux de les isoler sous forme de sel de sodium ou de potassium. Les acides libres peuvent ensuite être, si on le désire, libérés de leurs sels selon les techniques habituelles et être, si on le désire, retransformés en un autre sel avec une base.

Comme sels avec les bases, on peut citer les sels de sodium, de potassium ou les sels d'addition avec les bases azotées tels que les sels d'éthanolamine ou de lysine.

Les nouveaux produits de formule générale (II) présentent d'intéressantes propriétés pharmacologiques les rendant utiles dans le traitement des maladies nerveuses d'origine centrale.

Ils se sont en effet montrés actifs in vitro dans les tests d'affinité vis-à-vis des récepteurs du système nerveux central alors qu'ils sont beaucoup moins actifs vis-à-vis des sites récepteurs du système périphérique (pancréas, muscles lisses intestinaux). Ces propriétés sont mises en évidence d'une part dans des tests de liaison sur membranes de cerveau de souris et d'autre part dans des tests pharmacologiques de libération d'amylase pancréatique de rat et de contraction d'iléon de cobaye.

1) Tests de liaisons

Les produits selon l'invention sont testés dans des expériences de compétition vis-à-vis du ligand [$^3$H] BOC

[NLe[28,31]] CCK27-33 sur cortex de cerveau de cobaye et acini pancréatique de cobaye. Les protocoles expérimentaux de préparation des tissus et les conditions de liaisons sont analogues à ceux décrits par PELAPRAT et al., Life Sci. 37, 2489 (1985), DURIEUX et al., Biochem. Biophys. Res. Commun., 137, 1167 (1986). Les résultats sont exprimés par les constantes d'inhibition $K_I$ dans le tableau I. Le [$^3$H] BOC [NLe[28,31]] CCK27-33 a été utilisé à une concentration de 0,2 nM pour le cerveau et 0,5 nM pour le pancréas.

TABLEAU I

| Produit testé | Test de liaison Cortex de cobaye $K_I$ (en M) | Test de liaison acini pancréatiques $K_I$ (en M) | Facteur de sélectivité $S.f. = \frac{K_I \text{ pancréas}}{K_I \text{ cerveau}}$ |
|---|---|---|---|
| Exemple 5 | $5.10 \pm 0.88\ 10^{-9}$ | $910 \pm 140\ 10^{-9}$ | 179 |
| Exemple 7 | $6.3 \pm 1.23\ 10^{-9}$ | $1010 \pm 110\ 10^{-9}$ | 160 |
| Exemple 15 | $4.35 \pm 1.10\ 10^{-9}$ | $860 \pm 60\ 10^{-9}$ | 197 |
| Exemple 16 | $0.49 \pm 0.03\ 10^{-9}$ | $970 \pm 160\ 10^{-9}$ | 1970 |
| CCK8 (référence) | $0.30 \pm 0.07\ 10^{-9}$ | $0.91 \pm 0.26\ 10^{-9}$ | 3 |

2) Test de libération d'amylase pancréatique

L'induction de libération d'amylase par ces dérivés de la CCK8 a été testée sur les acini pancréatiques de cobayes préparés suivant la méthode décrite par PEIKIN et al., Am. J. Physiol., 235, E 743 (1978). Les acini sont incubés 30 minutes à 37°C en présence des produits à étudier à différentes doses suivant la méthode décrite par GARDNER et JACKSON, J. Physiol., 270, 439 (1977). L'activité amylasique est déterminée à l'aide du réactif PHADEBAS (Pharmacia) selon la méthode décrite par CESKA et al., Experientia, 25, 555 (1969).

Les résultats observés figurent dans le tableau II suivant. Les valeurs indiquées dans ce tableau représentent la moyenne de 3 expériences indépendantes, chacune effectuée en triplicate.

TABLEAU II

| Produit testé | Sécrétion d'amylase pancréatique par acini de cobaye | |
|---|---|---|
| | Activité agoniste $EC_{50}$ (M) | Activité antagoniste $EC_{50}$ (M) |
| Exemple 5 | $> 10^{-5}$ | - |
| Exemple 7 | $> 10^{-5}$ | - |
| Exemple 16 | $> 10^{-5}$ | - |
| CCK8 (référence) | $1.12 \pm 0.14\ 10^{-10}$ | - |

3) Test d'activité contracturante sur l'iléon de cobaye

Ce test est réalisé selon la méthode décrite par HUTCHINSON et al., Eur. J. Pharmacol., 69, 87 (1981). Des bandelettes d'iléon de cobaye de la partie terminale sont rapidement prélevées et fixées dans la cuve d'un capteur isométrique contenant 25 $cm^3$ d'une solution de Tyrode. La solution est maintenue à 37°C tout en faisant barboter bulle à bulle un gaz composé de 95% d'oxygène et de 5% de $CO_2$. Les composés à étudier sont testés dans les conditions décrites par RUIZ-GAYO et al., Peptides, 6, 415 (1985).

Les résultats observés sont reportés dans le tableau III. Les valeurs indiquées dans ce tableau pour l'activité agoniste représentent la moyenne (± e.s.m.) de 3 expériences indépendantes. L'activité antagoniste est mesurée après induction de la contraction au moyen de $3.10^{-9}$M de CCK8.

TABLEAU III

| Produit testé | Activité contracturante sur iléon de cobaye | |
|---|---|---|
| | Activité agoniste $EC_{50}$ (M) | Activité antagoniste $EC_{50}$ (M) |
| Exemple 5 | › $10^{-5}$ | - |
| Exemple 7 | › $10^{-5}$ | - |
| Exemple 16 | › $10^{-5}$ | - |
| CCK8 (référence) | 0.68 ± 0.02 $10^{-9}$ | - |

Les produits selon l'invention présentent une toxicité assez faible. Leur $DL_{50}$ chez la souris par voie intraveineuse est généralement comprise entre 50 et 100 mg/kg.

Sont particulièrement intéressants les produits de formule générale (II) dans laquelle X représente un atome d'hydrogène ou un radical tert-butyloxycarbonyle, A et D représentent Asp, Glu, Lys, Orn, Cys ou Hcy (sous la forme L ou D), R représente un atome d'hydrogène ou un radical $SO_3H$ (l'acide aminé correspondant étant sous la forme L ou D), B représente NLe ou Thr (sous la forme L), E représente NLe, Hcy (S-t-Bu) (sous la forme L), F représente Phe (sous la forme L) et Q représente un atome d'hydrogène, étant entendu que dans la formule générale (II), Trp et Asp sont sous forme L.

Sont plus particulièrement intéressants les produits suivants :

- BOC-DAsp-Tyr($SO_3$Na)-NLe-DLys-Trp-NLe-Asp(Na)-Phe-$NH_2$
- BOC-DGlu-Tyr($SO_3$Na)-NLe-DLys-Trp-NLe-Asp(Na)-Phe-$NH_2$
- BOC-γ-DGlu-Tyr-NLe-DLys-Trp-NLe-Asp-Phe-$NH_2$
- BOC-γ-DGlu-Tyr($SO_3$Na)-NLe-DLys-Trp-NLe-Asp(Na)-Phe-$NH_2$

Les exemples suivants, donnés à titre non limitatif, montrent comment l'invention peut être mise en pratique. Les exemples de référence se rapportent à la préparation des matières premières. Dans ces exemples, les acides aminés sont représentés par la forme abrégée conventionnelle. L'enchaînement peptidique représenté dans le texte par BOC-γ-DGlu-Tyr- correspond à l'amidification entre la fonction γ-carboxylique du résidu Glu et la fonction α aminée de la tyrosine. La fonction α-carboxylique du résidu Glu est dans ce cas soit libre, soit amidifiée avec la fonction ε-amine de la chaîne latérale de la lysine suivant la formule du composé. Les autres abréviations ont les significations suivantes :

BOC = tertiobutyloxycarbonyl

OBZ = benzyloxy
Z = benzyloxycarbonyl
Ac = acétyl
OMe = méthoxy
t-Bu = tertio-butyl
Suc = succinyl
TFA = acide trifluoroacétique
Nps = nitro-2 phénylsulfényl
Cha = cyclohexylalanine
ONp = nitro-4 phényloxy

Les spectres de masse obtenus par ionisation FAB ont été effectués sur un spectromètre magnétique Ion Tech Ltd à 6 kV avec un faisceau d'atomes de xénon accéléré à 8 kV.

EXEMPLE 1

BOC-Asp-Tyr-NLe-DLys-Trp-NLe-Asp-Phe-$NH_2$

On dissout 0,136 g de H-NLe-Asp(OBZ)-Phe-$NH_2$,TFA (préparé comme décrit à l'exemple de référence 13) dans 4 $cm^3$ de diméthylformamide contenant 0,035 $cm^3$ de triéthylamine. La solution est additionnée de 0,204 g de

BOC-Asp-Tyr-NLe-DLys-Trp-OH

(préparé comme décrit à l'exemple de référence 9), de 0,029 g de N-hydroxysuccinimide et 0,063 g de dicyclohexylcarbodiimide. Le mélange réactionnel est agité pendant une heure à 0°C puis pendant une nuit à une température voisine de 20°C. L'insoluble formé est éliminé par filtration et le filtrat est concentré à sec sous pression réduite (1 mm de mercure ; 0,13 kPa) à 40°C. Le résidu obtenu est précipité par un mélange d'acétate d'éthyle et d'éther diéthylique. On obtient ainsi 0,3 g de

BOC-Asp-Tyr-NLe-DLys-Trp-NLe-Asp(OBZ)-Phe-$NH_2$

sous forme d'un solide blanc fondant à 130-133°C.

Rf = 0,62 [silicagel ; chloroforme-méthanol (80-20 en volumes)].

Le déblocage de la fonction acide de l'acide aspartique peut être effectué de la manière suivante : A une solution de 0,25 g de

BOC-Asp-Tyr-NLe-DLys-Trp-NLe-Asp(OBZ)-Phe-$NH_2$,

obtenu comme décrit ci-avant, dans 4 $cm^3$ de méthanol, on ajoute 11 mg de palladium sur charbon actif (à 10% en poids) et on hydrogène le mélange réactionnel sous pression atmosphérique à une température voisine de 20°C. Le catalyseur est éliminé par filtration et le filtrat est concentré à sec sous pression réduite (20 mm de mercure ; 2.7 kPa) à 40°C. On obtient ainsi 0,216 g de

BOC-Asp-Tyr-NLe-DLys-Trp-NLe-Asp- Phe-$NH_2$.

Rf = 0,54 [silicagel : acétate d'éthyle-pyridine-acide acétique-eau (80-20-6-11 en volumes)].

Spectre de masse en ionisation FAB : m/e = 1182 $(MH)^+$

Analyse d'acides aminés : Asp = 1,89 ; NLe = 2 ; Tyr = 0,91 ; Phe = 0,98 ; Lys = 0,92.

EXEMPLE 2

BOC-Asp-Tyr($SO_3$Na)-NLe-DLys-Trp-NLe-Asp(Na)-Phe-$NH_2$

A une solution 216 mg de

BOC-Asp-Tyr-NLe-DLys-Trp-NLe- Asp-Phe-$NH_2$

(préparé comme décrit à l'exemple 1) dans 2 $cm^3$ de diméthylformamide anhydre et 6 $cm^3$ de pyridine anhydre, on ajoute 1,1 g de complexe $SO_3$-pyridine et agite le mélange réactionnel sous atmosphère d'azote pendant une nuit à une température voisine de 20°C. Après élimination des solvants sous pression réduite (1 mm de mercure ; 0,13 kPa) à 40°C, le résidu est repris par 5 $cm^3$ d'une solution aqueuse de bicarbonate de sodium et la suspension est agitée pendant une heure à une température voisine de 0°C en maintenant le pH à une valeur voisine de 7 au moyen d'une solution aqueuse saturée de bicarbonate de sodium. Une fraction de produit (300 mg) correspondant au produit en suspension est collectée par centrifugation. Une seconde fraction (100 mg) moins pure est obtenue par lyophilisation de la phase aqueuse et précipitation des sels minéraux par du méthanol. Ces deux fractions sont réunies et purifiées par chromatographie sur une collone de gel de silice (diamètre 2 cm) en éluant avec un mélange d'acétate d'éthyle, de pyridine, d'acide acétique et d'eau (60-20-6-11 en volumes). On obtient ainsi 66 mg de

BOC-Asp-Tyr($SO_3$Na)-NLe-DLys-Trp-NLe-Asp(Na)-Phe-$NH_2$

sous forme d'un solide blanc.

Rf = 0,23 [silicagel ; acétate d'éthyle-pyridine-acide acétique-eau (40-20-6-11 en volumes)].

Spectre de masse en ionisation FAB : m/e = 1306 $(MH)^+$

Analyse d'acides aminés : Asp = 1,91 ; NLe = 2,0 ; Tyr = 0,92 ; Phe = 0,96 ; Lys = 0,93.

En opérant comme décrit aux exemples 1 et 2 précédents mais à partir des matières premières convenables, ont été préparés :

EXEMPLE 3

BOC-Asp-Tyr($SO_3$Na)-NLe-DLys-Trp-NLe-Asp(Na)-$NH_2$

Rf = 0,28 [silicagel ; acétate d'éthyle-pyridine-acide acétique-eau (60-20-6-11 en volumes)].

Spectre de masse en ionisation FAB : m/e = 1160 $(MH)^+$

Analyse d'acides aminés : Asp = 2,02 ; NLe = 2,0 ; Tyr = 0,93 ; Lys = 0,94.

EXEMPLE 4

BOC-Glu-Tyr($SO_3$Na)-NLe-DLys-Trp-NLe-Asp(Na)-Phe-$NH_2$

Rf = 0,30 [silicagel ; acétate d'éthyle-pyridine-acide acétique-eau (60-20-6-11 en volumes)].

Spectre de masse en ionisation FAB : m/e = 1320 $(MH)^+$

Analyse d'acides aminés : Asp = 0,99 ; Glu = 0,95 ; NLe = 2,0 ; Tyr = 0,89 ; Phe = 1,03 ; Lys = 0,96.

EXEMPLE 5

BOC-DAsp-Tyr($SO_3$Na)-NLe-DLys-Trp-NLe-Asp(Na)-Phe-$NH_2$

Rf = 0,27 [silicagel ; acétate d'éthyle-pyridine-acide acétique-eau (60-20-6-11 en volumes)]. Spectre de masse en ionisation FAB : m/e = 1306 $(MH)^+$ Analyse d'acides aminés : Asp = 1,93 ; NLe = 2,0 ; Tyr = 0,93 ; Phe = 0,96 ; Lys = 0,93.

EXEMPLE 6

H-DAsp-Tyr($SO_3$Na)-NLe-DLys-Trp-NLe-Asp(Na)-Phe-$NH_2$

Rf = 0,20 [silicagel ; acétate d'éthyle-pyridine-acide acétique-eau (40-20-6-11 en volumes)]. Spectre de masse en ionisation FAB : m/e = 1206 $(MH)^+$ Analyse d'acides aminés : Asp = 2,03 ; NLe = 2,0 ; Tyr = 0,99 ; Phe = 1,01 ; Lys = 0,96.

EXEMPLE 7

BOC-DGlu-Tyr($SO_3$Na)-NLe-DLys-Trp-NLe-Asp(Na)-Phe-$NH_2$

Rf = 0,28 [silicagel ; acétate d'éthyle-pyridine-acide acétique-eau (60-20-6-11 en volumes)]. Spectre de masse en ionisation FAB : m/e = 1320 $(MH)^+$ Analyse d'acides aminés : Asp = 1,02 ; Glu = 0,97 ; NLe = 2,0 ; Tyr = 0,96 ; Phe = 1,03 ; Lys = 0,99.

EXEMPLE 8

BOC-DGlu-Tyr($SO_3$Na)-NLe-DOrn-Trp-NLe-Asp(Na)-Phe-$NH_2$

Rf = 0,40 [silicagel ; acétate d'éthyle-pyridine-acide acétique-eau (40-20-6-11 en volumes)]. Spectre de masse en ionisation FAB : m/e = 1306 $(MH)^+$ Analyse d'acides aminés : Asp = 1,02 ; Glu = 1,00 ; NLe = 2,0 ; Tyr = 0,94 ; Phe = 1,01 ; Orn = 0,96.

EXEMPLE 9

BOC-DLys-Tyr($SO_3$Na)-NLe-DAsp-Trp-NLe-Asp(Na)-Phe-$NH_2$

Rf = 0,32 [silicagel ; acétate d'éthyle-pyridine-acide acétique-eau (20-20-6-11) en volumes)]. Spectre de masse en ionisation FAB : m/e = 1306 $(MH)^+$ Analyse d'acides aminés : Asp = 1,97 ; NLe = 2,0 ; Tyr = 0,95 ; Phe = 1,03 ; Lys = 0,95.

EXEMPLE 10

BOC-DLys-Tyr($SO_3$Na)-NLe-DGlu-Trp-NLe-Asp(Na)-Phe-$NH_2$

Rf = 0,21 [silicagel ; acétate d'éthyle-pyridine-acide acétique-eau (80-20-6-11 en volumes)]. Spectre de masse en ionisation FAB : m/e = 1320 $(MH)^+$ Analyse d'acides aminés : Asp = 1,02 ; Glu = 1,04 ; NLe = 2,0 ; Tyr = 0,98 ; Phe = 1,01 ; Lys = 0,98.

EXEMPLE 11

S ———————— S

BOC-DCys-Tyr($SO_3$Na)-NLe-DCys-Trp-NLe-Asp(Na)-Phe-$NH_2$

Rf = 0,42 [silicagel ; acétate d'éthyle-pyridine-acide acétique-eau (40-20-6-11 en volumes)].
Spectre de masse en ionisation FAB : m/e = 1284 $(MH)^+$
Analyse d'acides aminés : Asp = 1,02 ; NLe = 2,0 ; Tyr = 0,93 ; Phe = 0,99 ; Cys = 1,76.

EXEMPLE 12

S ———————— S

BOC-DHcy-Tyr($SO_3$Na)-NLe-DHcy-Trp-NLe-Asp(Na)-Phe-$NH_2$

Rf = 0,34 [silicagel ; acétate d'éthyle-pyridine-acide acétique-eau (60-20-6-11 en volumes)].
Spectre de masse en ionisation FAB : m/e = 1256 $(MH)^+$
Analyse d'acides aminés :Asp = 1,03 ; NLe = 2,0 ; Hcy = 1,81 ; Tyr = 0,96 ; Phe = 1,02.

EXEMPLE 13

BOC-DAsp-Tyr-NLe-DLys-Trp-NLe-Asp-Phe-$NH_2$

Rf = 0,58 [silicagel ; acétate d'éthyle-pyridine-acide acétique-eau (80-20-6-11 en volumes)].
Spectre de masse en ionisation FAB : m/e = 1182 $(MH)^+$
Analyse d'acides aminés : Asp = 2,04 ; NLe = 2,0 ; Tyr = 0,94 ; Phe = 1,02 ; Lys = 0,97.

EXEMPLE 14

H-DGlu-Tyr($SO_3$Na)-NLe-DLys-Trp-NLe-Asp(Na)-Phe-$NH_2$

Rf = 0,22 [silicagel ; acétate d'éthyle-pyridine-acide acétique-eau (60-20-6-11 en volumes)].
Spectre de masse en ionisation FAB : m/e = 1220 $(MH)^+$
Analyse d'acides aminés : Asp = 1,01 ; Glu= 1,03 ; NLe = 2,0 ; Tyr = 0,93 ; Phe = 1,01 ; Lys = 0,98.

EXEMPLE 15

BOC-γ-DGlu-Tyr-NLe-DLys-Trp-NLe-Asp-Phe-$NH_2$

On dissout 0,235 g de H-NLe-Asp(OBZ)-Phe-$NH_2$, TFA (préparé comme décrit à l'exemple de référence 13) dans 6 $cm^3$ de diméthylformamide contenant 0,054 $cm^3$ de triéthylamine. La solution est additionnée de 0,313 g de

BOC-γ-DGlu-Tyr-NLe-DLys-Trp-OH

(préparé comme décrit dans l'exemple de référence 17), de 0,070 g de N-hydroxysuccinimide et de 0,095 g de dicyclohexylcarbodiimide. Le mélange réactionnel est agité pendant une heure à 0°C puis pendant une nuit à une température voisine de 20°C. L'insoluble formé est éliminé par filtration et le filtrat est concentré à sec

sous pression réduite (1 mm de mercure ; 0,13 kPa) à 40°C. Le résidu obtenu est précipité par un mélange d'acétate d'éthyle et d'éther diéthylique. On obtient ainsi 0,48 g de

BOC-γ-DGlu-Tyr-NLe-DLys-Trp-NLe-Asp(OBZ)-Phe-$NH_2$

sous forme d'un solide blanc.

Rf = 0,66 [silicagel ; acétate d'éthyle-pyridine-acide acétique-eau (120-20-6-11 en volumes)].

Le déblocage de la fonction acide de l'acide aspartique peut être effectué de la manière suivante : A une solution de 0,47 g de

BOC-γ-DGlu-Tyr-NLe-DLys-Trp-NLe-Asp(OBZ)-$NH_2$,

obtenu comme décrit ci-avant, dans 5 $cm^3$ de méthanol et 5 $cm^3$ de diméthylformamide, on ajoute 40 mg de palladium sur charbon actif (à 10% en poids) et on hydrogène pendant 2 heures le mélange réactionnel sous pression atmosphérique à une température voisine de 20°C. Le catalyseur est éliminé par filtration et le filtrat est concentré à sec sous pression réduite (20 mm de mercure ; 2,7 kPa) à 40°C. On obtient ainsi 0,44 g de

BOC-γ-DGlu-Tyr-NLe-DLys-Trp-NLe-Asp-Phe-$NH_2$

sous forme d'un produit solide blanc fondant à 195-197°C.

Rf = 0,30 [silicagel ; acétate d'éthyle-pyridine-acide acétique-eau (120-20-6-11 en volumes)].

Spectre de masse en ionisation FAB : m/e = 1196 $(MH)^+$

Analyse d'acides aminés : Asp = 2,03 ; Glu = 1,01 ; NLe = 2,0 ; Tyr = 0,93 ; Phe = 0,99 ; Lys = 0,95.

<u>EXEMPLE 16</u>

BOC-γ-DGlu-Tyr($SO_3$Na)-NLe-DLys-Trp-NLe-Asp(Na)-Phe-$NH_2$

A une solution de 0,23 g de

BOC-γ-DGlu-Tyr-NLe-DLys- Trp-NLe-Asp-Phe-$NH_2$

(préparé comme décrit à l'exemple 15) dans 2 $cm^3$ de diméthylformamide anhydre et 6 $cm^3$ de pyridine anhydre, on ajoute 1,3 g de complexe $SO_3$-pyridine et agite le mélange sous atomsphère d'azote pendant une nuit à une température voisine de 20°C. Après élimination des solvants sous pression réduite (1 mm de mercure ; 0,13 kPa) à 40°C, le résidu est repris par 5 $cm^3$ d'une solution aqueuse saturée de bicarbonate de sodium et la solution est agitée pendant une heure à une température voisine de 0°C en maintenant le pH à une valeur voisine de 7 au moyen d'une solution aqueuse saturée de bicarbonate de sodium. Une fraction de produit (250 mg) correspondant au produit en suspension est obtenue par centrifugation. Une seconde fraction (120 mg) moins pure est obtenue par lyophilisation de la phase aqueuse et précipitation des sels minéraux par du méthanol. Ces deux fractions sont réunies et purifiées par chromatographie sur une colonne de gel de silice (diamètre: 2 cm) en éluant avec un mélange d'acétate d'éthyle, de pyridine, d'acide acétique et d'eau (65-20-6-11 en volumes). On obtient ainsi 100 mg de

BOC-γ-DGlu-Tyr($SO_3$Na)-NLe-DLys-Trp-NLe-Asp(Na)-Phe-$NH_2$

sous forme d'un solide blanc.

Rf = 0,45 [silicagel ; butanol-acide acétique-eau (4-1-1 en volumes)].

Spectre de masse en ionisation FAB : m/e = 1320 $(MH)^+$

Analyse d'acides aminés : Asp = 0,92 ; Glu = 0,94 ; NLe = 2,0 ; Tyr =0,94 ; Phe = 1,01 ; Lys = 0,99.

EXEMPLE DE REFERENCE 1

BOC-DLys(Z)-Trp-OMe

A une solution refroidie à 0°C de 2,64 g de chlorhydrate de tryptophanate de méthyle dans 120 $cm^3$ d'un mélange de tétrahydrofuranne et de chloroforme (60-40 en volumes), on ajoute 1,69 $cm^3$ de thriéthylamine puis 4,6 g de BOC-DLys(Nε-Z)-OH et 1,8 g d'hydroxy-1 benzotriazole, 2,73 g de dicyclohexylcarbodiimide et agite le mélange réactionnel sous atmosphère d'azote pendant une heure à 0°C puis pendant une nuit à une température voisine de 20°C. Le solide formé est éliminé par filtration et le filtrat est concentré à sec sous pression réduite (20 mm de mercure ; 2,7 kPa) à une température voisine de 40°C. L'huile obtenue est reprise par de l'acétate d'éthyle. La solution obtenue est lavée avec une solution aqueuse à 10% d'acide citrique puis avec une solution aqueuse saturée de bicarbonate de sodium puis enfin avec une solution aqueuse saturée de chlorure de sodium jusqu'à neutralité. La phase organique est séchée sur sulfate de sodium, filtrée et évaporée à sec sous pression réduite (20 mm de mercure ; 2,7 kPa) à une température voisine de 40°C. Le résidu obtenu est recristallisé dans un mélange d'acétate d'éthyle et d'hexane. On obtient ainsi 6,07 g de BOC-DLys(Z)-Trp-OMe fondant à 68-70°C.

Rf = 0,53 [silicagel ; chloroforme-méthanol (9-1 en volumes)].

EXEMPLE DE REFERENCE 2

BOC-NLe-DLys(Z)-Trp-OMe

On dissout 7,5 g de BOC-DLys(Z)-Trp-OMe (préparé comme décrit à l'exemple de référence 1) dans un mélange refroidi à 0°C de chlorure de méthylène, d'acide trifluoroacétique et d'anisole (respectivement 20 $cm^3$, 20 $cm^3$ et 1 $cm^3$) et agite le mélange réactionnel sous azote pendant 45 minutes à 0°C puis pendant 45 minutes à température ambiante. Les solvents sont ensuite évaporés sous pression réduite (1 mm de mercure ; 0,13 kPa) à une température voisine de 40°C. Le solide amorphe résultant est repris par 100 $cm^3$ d'un mélange de chloroforme et de tétrahydrofuranne (50-50 en volumes) refroidi à 0°C et le mélange est additionné successivement de 1,81 $cm^3$ de triéthylamine, 3 g de N-tert-butyloxycarbonyl-norleucine, 1,98 g d'hydroxy-1 benzotriazole et 2,67 g de dicyclohexylcarbodiimide. Le mélange réactionnel est agité sous azote pendant une heure à 0°C puis pendant une nuit à une température voisine de 20°C. Le solide formé est éliminé par filtration et le filtrat est concentré à sec sous pression réduite (20 mm de mercure ; 2,7 kPa) à une température voisine de 40°C. L'huile obtenue est reprise par de l'acétate d'éthyle. La solution obtenue est lavée avec une solution aqueuse à 10% d'acide citrique puis avec une solution aqueuse saturée de bicarbonate de sodium puis enfin avec une solution aqueuse saturée de chlorure de sodium jusqu'à neutralité. La phase organique est séchée sur sulfate de sodium, filtrée et évaporée à sec sous pression réduite (20 mm de mercure ; 2,7 kPa) à une température voisine de 40°C. Le résidu obtenu est recristallisé dans un mélange d'acétate d'éthyle et d'éther diéthylique. On obtient ainsi 7,75 g de BOC-NLe-DLys(Z)-Trp-OMe fondant à 80-82°C.

Rf = 0,35 [silicagel ; chloroforme-méthanol (95-5 en volumes)].

EXEMPLE DE REFERENCE 3

H-NLe-DLys(Z)-Trp-OMe,TFA

On dissout 6,2 g de BOC-NLe-DLys(Z)-Trp-OMe (préparé comme décrit à l'exemple de référence 2) dans un mélange refroidi à 0°C de chlorure de méthylène, d'acide trifluoroacétique et d'anisole (respectivement 15 $cm^3$, 15 $cm^3$ et 0,8 $cm^3$) et agite sous azote le mélange réactionnel pendant 45 minutes à une température voisine de 20°C. Les solvants sont ensuite évaporés sous pression réduite (1 mm de mercure ; 0,13 kPa) à 40°C et le résidu obtenu est purifié par chromatographie flash sur gel de silice en éluant avec un mélange de chloroforme et de méthanol (95-5 en volumes). On obtient ainsi 5,68 g de H-NLe-DLys(Z)-Trp-OMe,TFA.

Rf = 0,49 [silicagel ; chloroforme-méthanol (80-20 en volumes)].

EXEMPLE DE REFERENCE 4

BOC-Tyr-NLe-DLys(Z)-Trp-OMe

A une soluton refroidie à 0°C de 3,46 g de H-NLe-DLys(Z)-Trp-OMe,TFA (préparé comme décrit à l'exemple de référence 3) dans 30 $cm^3$ de chloroforme, on ajoute successivement 0,68 $cm^3$ de triéthylamine, 1,38 g de N-tert-butyloxycarbonyl-tyrosine, 0,75 g d'hydroxy-1 benzotriazole et 1,01 g de dicyclohexylcarbodiimide. Le mélange réactionnel est agité sous azote pendant une heure à 0°C puis pendant une nuit à une température voisine de 20°C. L'insoluble formé est éliminé par filtration et le filtrat est concentré à sec sous pression réduite (20 mm de mercure ; 2,7 kPa) à 40°C. Le résidu huileux obtenu est purifié par chromatographie flash sur gel

de silice en éluant avec un mélange de chloroforme et de méthanol (97-3 en volumes). On obtient ainsi 3,3 g de BOC-Tyr-NLe-DLys(Z)-Trp-OMe fondant à 152-154°C.

Rf = 0,46 [silicagel ; chloroforme-méthanol (90-10 en volumes)].

EXEMPLE DE REFERENCE 5

H-Tyr-NLe-DLys(Z)-Trp-OMe,TFA

On dissout 2,7 g de BOC-Tyr-NLe-DLys(Z)-Trp-OMe (préparé comme décrit à l'exemple de référence 4) dans un mélange refroidi à 0°C de chlorure de méthylène, d'acide trifluoroacétique et d'anisole (respectivement 5 cm³, 5 cm³ et 0,2 cm³) et agite le mélange réactionnel sous azote pendant 45 minutes à une température voisine de 0°C et 45 minutes à une température voisine de 20°C. Les solvants sont ensuite évaporés sous pression réduite (20 mm de mercure ; 2,7 kPa) à 40°C et le résidu obtenu est précipité par de l'éther diéthylique. On obtient ainsi 2,3 g de H-Tyr-NLe-DLys(Z)-Trp-OMe,TFA.

Rf = 0,58 [silicagel ; chloroforme-méthanol (80-20 en volumes)].

EXEMPLE DE REFERENCE 6

BOC-Asp(OBZ)-Tyr-NLe-DLys(Z)-Trp-OMe

A une solution refroidie à 0°C de 1,15 g de H-Tyr-NLe-DLys(Z)-Trp-OMe (préparé comme décrit à l'exemple de référence 5) dans 8 cm³ de diméthylformamide, on ajoute successivement 0,41 cm³ de N,N-diisopropylamine, 0,64 g de BOX-Asp(OBZ)-ONp et 0,186 g d'hydroxy-1 benzotriazole et agite le mélange réactionnel sous atmosphère d'azote pendant une heure à 0°C puis pendant deux heures à une température voisine de 20°C. Les solvants sont ensuite évaporés sous pression réduite (1 mm de mercure ; 0,13 kPa) à 40°C. Le residu huileux est trituré avec de l'éther diéthylique et le solide obtenu est recristallisé dans un mélange d'acétate d'éthyle et d'hexane. On obtient ainsi 1,2 g de BOC-Asp(OBZ)-Tyr-NLe-DLys(Z)-Trp-OMe sous forme d'un solide jaune fondant à 182-184°C.

Rf = 0,72 [silicagel ; chloroforme-méthanol (80-20 en volumes)].

EXEMPLE DE REFERENCE 7

BOC-Asp-Tyr-NLe-DLys-Trp-OMe

A une solution de 0,6 g de BOC-Asp(OBZ)-Tyr-Nle-DLys(Z)-Trp-OMe (préparé comme décrit à l'exemple de référence 6) dans 20 cm³ de méthanol, on ajoute 0,12 g de palladium sur charbon actif (10 % en poids) et hydrogène le mélange sous pression atmosphérique à une température voisine de 20°C pendant 2 heures. Après élimination du catalyseur par filtration, le filtrat est concentré à sec sous pression réduite (20 mm de mercure ; 2,7 kPa) à 40°C. On obtient ainsi 4,6 g de BOC-Asp-Tyr-NLe-DLys-Trp-OMe.

Rf = 0,36 [silicagel ; acétate d'éthyle-pyridine-acide acétique-eau (40-20-6-11 en volumes)].

EXEMPLE DE REFERENCE 8

BOC-Asp-Tyr-NLe-DLys-Trp-OMe

A une solution refroidie à −40°C et conservée sous atmosphère d'azote de 0,74 g de BOC-Asp-Tyr-NLe-DLys-Trp-OMe (préparé comme décrit à l'exemple de référence 7) dans 145 cm³ de diméthylformamide contenant 0,246 cm³ de triéthylamine, on ajoute goutte à goutte sous agitation en l'espace d'une heure une solution de 0,248 cm³ de diphénylphosphorylazide dans 5 cm³ de diméthylformamide et poursuit l'agitation pendant 48 heures à une température voisine de −25°C puis pendant 48 heures à 0°C. Durant toute la réaction, le pH apparent du milieu réactionnel est maintenu entre 7 et 7,5 par addition de triéthylamine. Le mélange réactionnel est ensuite concentré à sec sous pression réduite (1 mm de mercure ; 0,13 kPa) à 40°C. L'huile obtenue est purifiée par chromatographie flash sur gel de silice en éluant avec un mélange de chloroforme et de méthanol (95-5 en volumes). On obtient ainsi 0,37 g de solide blanc fondant à 172-174°C.

Rf = 0,31 [silicagel ; chloroforme-méthanol (90-10 en volumes)].

Spectre de masse en ionisation FAB : m/e = 806 $(MH)^+$

EXEMPLE DE REFERENCE 9

BOC-Asp-Tyr-NLe-DLys-Trp-OH

A une solution refroidie à environ 0°C de 0,33 g de

BOC-Asp-Tyr-NLe-DLys-Trp-OMe

(préparé comme décrit à l'exemple de référence 8) dans 4,5 $cm^3$ de méthanol, on ajoute 0,8 $cm^3$ d'une solution aqueuse de soude 1N et agite le mélange réactionnel sous azote pendant 2 heures à 0°C puis pendant une nuit à une température voisine de 20°C. Le mélange réactionnel est concentré à sec sous pression réduite (20 mm de mercure ; 2,7 kPa) à 40°C et le résidu est dissous dans 5 $cm^3$ d'eau distillée. La solution obtenue est extraite à l'éther diéthylique et éliminée ; la phase aqueuse est acidifiée à 0°C avec une solution aqueuse d'acide chlorhydrique 1N, puis extraite à l'acétate d'éthyle. La solution organique est lavée avec une solution aqueuse saturée de chlorure de sodium puis séchée sur sulfate de sodium et filtrée. Le filtrat est concentré à sec sous pression réduite (20 mm de mercure ; 2,7 kPa) à 40°C. On obtient ainsi 0,24 g de

BOC-Asp-Tyr-NLe-DLys-Trp-OH

fondant à 205-207°C.

Rf = 0,37 [silicagel ; acétate d'éthyle-pyridine-acide acétique-eau (80-20-6-11 en volumes)].

EXEMPLE DE REFERENCE 10

BOC-Asp(OBZ)-Phe-$NH_2$

A une solution refroidie à 0°C de 1,07 g de trifluoroacétate de phénylalanine dans 10 $cm^3$ de diméthylformamide contenant 0,6 $cm^3$ de triéthylamine, on ajoute 1,86 g de BOC-Asp(OBZ)-ONp et le mélange réactionnel est agité à une température voisine de 20°C pendant 24 heures. Le mélange réactionnel est concentré à sec sous pression réduite (1 mm de mercure ; 0,13 kPa) à 40°C. Le résidu obtenu est repris par de l'acétate d'éthyle. La solution obtenue est lavée avec une solution aqueuse à 10% d'acide citrique puis avec une solution aqueuse saturée de bicarbonate de sodium puis enfin avec une solution aqueuse saturée de chlorure de sodium jusqu'à neutralité. La phase organique est séchée sur sulfate de sodium, filtrée et évaporée à sec sous pression réduite (20 mm de mercure ; 2,7 kPa) à une température voisine de 40°C. Le résidu obtenu est recristallisé dans un mélange d'acétate d'éthyle et d'hexane. On obtient ainsi 1,47 g de BOC-Asp(OBZ)-Phe-$NH_2$, sous forme d'un solide blanc fondant à 137-138°C.

Rf = 0,38 [silicagel ; chloroforme-méthanol (95-5 en volumes)].

EXEMPLE DE REFERENCE 11

H-Asp(OBZ)-Phe-$NH_2$, TFA

On dissout 1 g de BOC-Asp(OBZ)-Phe-$NH_2$ (préparé comme décrit à l'exemple de référence 10) dans un mélange refroidi à 0°C de chlorure de méthylène et d'acide trifluoroacétique (2,5 $cm^3$, 2,5 $cm^3$) et agite le mélange réactionnel pendant 45 minutes à 0°C et pendant 45 minutes à une température voisine de 20°C. Les solvants sont ensuite évaporés sous pression réduite (20 mm de mercure ; 2,7 kPa) à 40°C. Le résidu obtenu est concrété avec de l'éther diéthylique. On obtient ainsi 1,85 g de H-Asp(OBZ)-Phe-$NH_2$,TFA.

Rf = 0,23 [silicagel ; chloroforme-méthanol (90-10 en volumes)].

EXEMPLE DE REFERENCE 12

BOC-NLe-Asp(OBZ)-Phe-$NH_2$

A une solution refroidie à 0°C de 1,83 g de H-Asp(OBZ)-Phe-$NH_2$, TFA (préparé comme décrit à l'exemple de référence 11) dans 20 $cm^3$ de diméthylformamide contenant 1,3 $cm^3$ de N,N -diisopropyléthylamine, on ajoute 1,48 g de BOC-NLe-ONp et 0,578 g d'hydroxy-2 benzotriazole et agite le mélange réactionnel pendant 30 minutes à 0°C puis pendant une nuit à une température voisine de 20°C. Après évaporation du solvant sous

pression réduite (1 mm de mercure ; 0,13 kPa) à 40°C, le résidu huileux obtenu est précipité par un mélange d'acétate d'éthyle et d'hexane. On obtient ainsi 1,98 g de BOC-NLe-Asp(OBZ)-Phe-$NH_2$ sous forme d'un solide blanc fondant à 134-136°C.

Rf = 0,61 [silicagel ; chloroforme-méthanol (90-10 en volumes)].

EXEMPLE DE REFERENCE 13

H-NLe-Asp(OBZ)-Phe-$NH_2$,TFA

On dissout 0,151 g de BOC-NLe-Asp(OBZ)-Phe-$NH_2$ (préparé comme décrit à l'exemple de référence 12) dans un mélange refroidi à 0°C de chlorure de méthylène et d'acide trifluoroacétique (respectivement 1 $cm^3$ et 1 $cm^3$) et agite le mélange réactionnel pendant 45 minutes à 0°C et 45 minutes à une température voisine de 20°C. Le mélange est concentré à sec sous pression réduite (20 mm de mercure ; 2,7 kPa) à 40°C. On obtient ainsi 0,136 g de H-NLe-Asp(OBZ)-Phe-$NH_2$, TFA.

Rf = 0,30 [silicagel ; chloroforme-éthanol (80-20 en volumes)].

EXEMPLE DE REFERENCE 14

BOC-γ-DGlu(α-OBZ)-Tyr-NLe-DLys(Z)-Trp-$OCH_3$

A une solution refroidie à 0°C de 1,29 g de H-Tyr-NLe-DLys (Z)-Trp-$OCH_3$ (préparé comme décrit dans l'exemple de référence 5) dans 10 $cm^3$ de diméthylformamide, on ajoute successivement 0,204 $cm^3$ de triéthylamine, 0,49 g de BOC-DGlu(α-OBZ)-OH, 0,17 g d'hydroxysuccinimide et 0,30 g de dicyclohexylcarbodiimide. Le mélange réactionnel est agité sous azote pendant une heure à 0°C puis une nuit à une température voisine de 20°C. L'insoluble formé est éliminé par filtration et le filtrat est concentré à sec sous pression réduite (20 mm de mercure ; 2,7 kPa) à 40°C. Le résidu huileux est purifié par précipitation par de l'acétate d'éthyle pour conduire à 1,3 g de BOC-γ-DGlu(α-OBZ)-Tyr-NLe-DLys(Z)-Trp-$OCH_3$ fondant à 110-113°C.

Rf = 0,48 [silicagel ; chloroforme-méthanol (90-10 en volumes)].

EXEMPLE DE REFERENCE 15

BOC-γ-DGlu-Tyr-NLe-DLys-Trp-$OCH_3$

A une solution de 1,27 g de BOC-γ-DGlu(α-OBZ)-Tyr-NLe-DLys (Z)-Trp-OMe (préparé comme décrit dans l'exemple de référence 14) dans 30 $cm^3$ de méthanol, on ajoute 0,16 g de palladium sur charbon actif (10% en poids) et hydrogène le mélange sous pression atmosphérique à une température voisine de 20°C pendant 3 heures. Après élimination du catalyseur par filtration, le filtrat est concentré à sec sous pression réduite (20 mm de mercure ; 2,7 kPa) à 40°C. On obtient ainsi 0,99 g de BOC-γ-DGlu-Tyr-NLe-DLys-Trp-$OCH_3$ fondant à 155-157°C.

Rf = 0,37 [silicagel ; chloroforme-méthanol (70-30 en volumes)].

EXEMPLE DE REFERENCE 16

BOC-γ-DGlu-Tyr-NLe-DLys-Trp-$OCH_3$

A une solution refroidie à −40°C et conservée sous atmosphère d'azote de 0,86 g de BOC-γ-DGlu-Tyr-NLe-DLys-Trp-$OCH_3$ (préparé comme décrit dans l'exemple de référence 15) dans 175 $cm^3$ de diméthylformamide contenant 0,282 $cm^3$ de triéthylamine, on ajoute goutte à goutte sous agitation en l'espace d'une heure, une solution de 0,258 $cm^3$ de diphénylphosphorylazide dans 5 $cm^3$ de diméthylformamide et poursuit l'agitation pendant 48 heures à une température voisine de −25°C puis pendant 48 heures à 0°C. Durant toute la réaction, le pH apparent du milieu réactionnel est maintenu entre 7 et 7,5 par addition de triéthylamine. Le mélange réactionnel est ensuite concentré à sec sous pression réduite (1 mm de mercure ; 0,13 kPa) à 40°C. L'huile obtenue est purifiée par chromatographie flash sur gel de silice en éluant avec un mélange de chloroforme et de méthanol (95-5 en volumes). On obtient ainsi 0,59 g de solide blanc fondant à 170-172°C.

Rf = 0,65 [silicagel ; chloroforme-méthanol (9-1 en volumes)].

Spectre de masse en ionisation FAB : m/e = 820 $(MH)^+$

EXEMPLE DE REFERENCE 17

BOC-γ-DGlu-Tyr-NLe-DLys-Trp-OH

A une solution refroidie à environ 0°C de 0,45 g de

BOC-γ-DGlu-Tyr-NLe-DLys-Trp-$OCH_3$

(préparé comme décrit à l'exemple de référence 16) dans 6 $cm^3$ de méthanol, on ajoute 1,1 $cm^3$ d'une solution aqueuse de soude 1N et agite le mélange réactionnel sous azote pendant 2 heures à 0°C et une nuit à une température voisine de 20°C. Le mélange réactionnel est concentré à sec sous pression réduite (20 mm de mercure ; 2,7 kPa) à 40°C et le résidu est dissous dans 5 $cm^3$ d'eau distillée. La solution obtenue est extraite à l'éther diéthylique et éliminée ; la phase aqueuse est acidifiée à 0°C avec une solution aqueuse d'acide chlorhydrique 1N, puis extraite à l'acétate d'éthyle. La solution organique est lavée avec une solution aqueuse saturée de chlorure de sodium puis séchée sur sulfate de sodium et filtrée. Le filtrat est concentré à sec sous pression réduite (20 mm de mercure ; 2,7 kPa) à 40°C. On obtient ainsi 0,31 g de

BOC-γ-DGlu-Tyr-NLe-DLys-Trp-OH,

solide blanc fondant à 181-182°C.

Rf = 0,30 [silicagel ; acétate d'éthyle-pyridine-acide acétique-eau (120-20-6-11 en volumes)].

La présente invention concerne également les médicaments constituées par un produit de formule générale (II), sous forme libre ou sous forme de sel d'addition avec un acide ou une base pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou sous forme de patches.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

En thérapeutique humaine, les produits selon l'invention sont particulièrement utiles dans le traitement des affections du système nerveux central dans lesquelles se produit un dysfonctionnement des voies dopaminergiques par exemple dans la maladie de Parkinson, les chorées, la schizophrénie, les dyskinésies tardives ou les épisodes maniaco-dépressifs.

Les doses dépendent de l'effet recherché et de la durée du traitement ; elles sont généralement comprises entre 2 et 10 mg par jour par voie parentérale pour un adulte en une ou plusieurs prises.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre des compositions selon l'invention.

EXEMPLE

On prépare une solution injectable contenant 1 mg de produit actif ayant la composition suivante :

- BOC-Asp-Tyr($SO_3Na$)-Nle-DLys-Trp-NLe-Asp(Na)-Phe-$NH_2$ ...... 1,03 mg

ou

- BOC-γ-DGlu-Tyr($SO_3Na$)-Nle-DLys-Trp-NLe-Asp(Na)-Phe-$NH_2$ ... 1,03 mg
- eau distillée ...................................... q.s.p. 2 cm3

**Revendications**

**Revendications pour les Etats Contractants : AT BE CH DE FR GB IT LI LU NL SE**

1. Nouveaux peptides dérivés de la CCK8, caractérisés en ce qu'ils répondent à la formule générale :

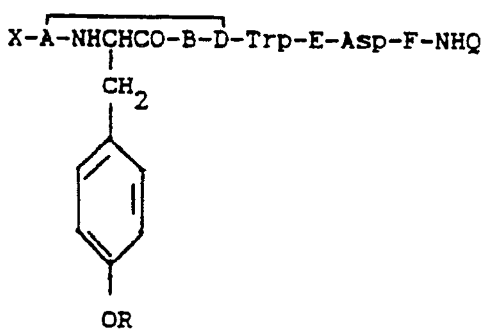

(II)

dans laquelle X représente un atome d'hydrogène ou bien un radical protecteur de la fonction amine du type acyle ou du type uréthane,

R représente un atome d'hydrogène ou un radical $—SO_3H$,

A et D sont définis comme suit :

a) ou bien A et D sont différents et représentent un radical de formule générale :

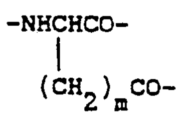 (III) ou 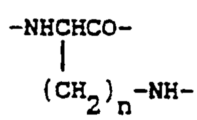 (IV)

dans lesquelles m et n représentent des nombres entiers compris entre 1 et 5, étant entendu que lorsque A représente un radical de formule générale (III) D représente un radical de formule générale (IV) et réciproquement et que A et D forment ensemble avec le reste de la molécule un cycle par amidification de leurs chaînes latérales,

b) ou bien A et D sont identiques ou différents et représentent un radical de formule générale :

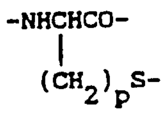 (V) ou 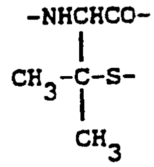 (VI)

dans lesquelles p représente un nombre entier égal à 1 ou 2, étant entendu que A et D forment ensemble avec le reste de la molécule un cycle par formation d'un pont disulfure entre les atomes de soufre et leurs chaînes latérales,

B et E, identiques ou différents, représentent un reste méthionine, nor-leucine, leucine, sérine, thréonine, allo-thréonine ou bien un reste cystéine ou homo-cystéine dont les fonctions OH ou SH peuvent être libres ou protégées

i) par un radical alcoyle contenant 1 à 6 atomes de carbone en chaîne droite ou ramifiée ou

ii) par un radical phényle non substitué ou substitué par un ou plusieurs atomes de fluor ou

iii) par un radical benzyle non substitué ou substitué par un ou plusieurs atomes de fluor ou

iv) par un radical alcoylcarbonyle dont la partie alcoyle contient 1 à 5 atomes de carbone en chaîne droite ou ramifiée ou bien benzoyle, phénylacétyle ou benzhydrylcarbonyle dont les parties phényle peuvent être non substituées ou substituées par un ou plusieurs atomes de fluor,

F représente une liaison de valence ou un reste phénylalanine, tyrosine O-méthylée ou cyclohexylalanine et

Q représente un atome d'hydrogène ou un radical alcoyle contenant 1 à 6 atomes de carbone en chaîne droite ou ramifiée non substitué ou substitué par un ou plusieurs atomes de fluor, ou un radical phényle ou phénylalcoyle dont la partie alcoyle contient 1 à 3 atomes de carbone en chaîne droite ou ramifiée et dont la partie phényle peut être non substituée ou substituée par un ou plusieurs atomes de fluor, étant entendu que les différents acides aminés entrant dans la définition des produits selon l'invention se présentent sous la forme D, L ou DL, ainsi que leurs sels pharmaceutiquement acceptables.

2. Procédé de préparation de peptides selon la revendication 1, caractérisé en ce que l'on fait réagir un acide de formule générale :

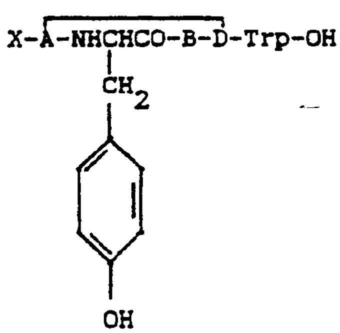 (VII)

dans laquelle les symboles sont définis comme à la revendication 1, sur une amine de formule générale :

H-E-Asp-F-NHQ (VIII)

dans laquelle les symboles sont définis comme à la revendication 1, suivie d'une sulfatation lorsqu'on veut obtenir un produit de formule générale (II) selon la revendication 1, dans laquelle R représente un radical $SO_3H$, puis isole le produit obtenu, soit à l'état libre soit à l'état de sel et le transforme, si on le désire, en un produit se présentant à l'état de sel ou à l'état libre.

3. Composition pharmaceutique, caractérisée en ce qu'elle contient au moins un produit de formule générale (II) défini comme à la revendication 1, en association avec un ou plusieurs diluants ou adjuvants compatibles et pharmaceutiquement acceptables.

**Revendication pour les Etats contractants suivants : ES GR**

1. Procédé de préparation de nouveaux peptides dérivés de la CCK8, de formule générale :

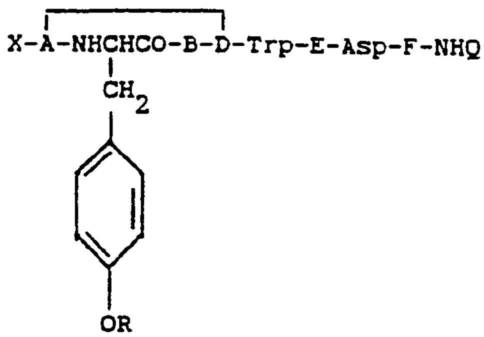

(II)

dans laquelle X représente un atome d'hydrogène ou bien un radical protecteur de la fonction amine du type acyle ou du type uréthane,

R représente un atome d'hydrogène ou un radical $-SO_3H$,

A et D sont définis comme suit :

a) ou bien A et D sont différents et représentent un radical de formule générale :

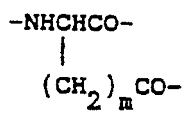 (III) ou 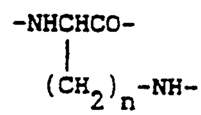 (IV)

dans lesquelles m et n représentent des nombres entiers compris entre 1 et 5, étant entendu que lorsque A représente un radical de formule générale (III) D représente un radical de formule générale (IV) et réciproquement et que A et D forment ensemble avec le reste de la molécule un cycle par amidification de leurs chaînes latérales,

b) ou bien A et D sont identiques ou différents et représentent un radical de formule générale :

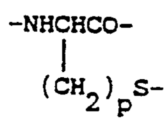 (V) ou 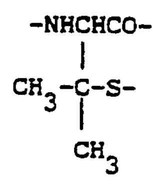 (VI)

dans lesquelles p représente un nombre entier égal à 1 ou 2, étant entendu que A et D forment ensemble avec le reste de la molécule un cycle par formation d'un pont disulfure entre les atomes de soufre et leurs chaînes latérales,

B et E, identiques ou différents, représentent un reste méthionine, nor-leucine, leucine, sérine, thréonine, allo-thréonine ou bien un reste cystéine ou homo-cystéine dont les fonctions OH ou SH peuvent être libres ou protégées

i) par un radical alcoyle contenant 1 à 6 atomes de carbone en chaîne droite ou ramifiée ou

ii) par un radical phényle non substitué ou substitué par un ou plusieurs atomes de fluor ou

iii) par un radical benzyle non substitué ou substitué par un ou plusieurs atomes de fluor ou

iv) par un radical alcoylcarbonyle dont la partie alcoyle contient 1 à 5 atomes de carbone en chaîne droite ou ramifiée ou bien benzoyle, phénylacétyle ou benzhydrylcarbonyle dont les parties phényle peuvent être non substituées ou substituées par un ou plusieurs atomes de fluor,

F représente une liaison de valence ou un reste phénylalanine, tyrosine O-méthylée ou cyclohexylalanine et

Q représente un atome d'hydrogène ou un radical alcoyle contenant 1 à 6 atomes de carbone en chaîne

droite ou ramifiée non substitué ou substitué par un ou plusieurs atomes de fluor, ou un radical phényle ou phénylalcoyle dont la partie alcoyle contient 1 à 3 atomes de carbone en chaîne droite ou ramifiée et dont la partie phényle peut être non substituée ou substituée par un ou plusieurs atomes de fluor, étant entendu que les différents acides aminés entrant dans la définition des produits selon l'invention se présentent sous la forme D, L ou DL, ainsi que leurs sels pharmaceutiquement acceptables, caractérisé en ce que l'on fait réagir un acide de formule générale :

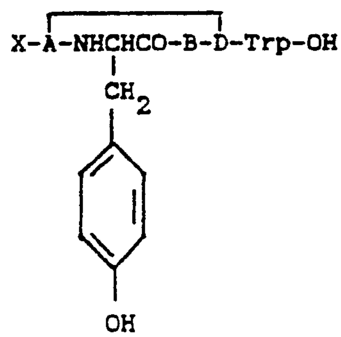

(VII)

dans laquelle les symboles sont définis comme à la revendication 1, sur une amine de formule générale :

H-E-Asp-F-NHQ (VIII)

dans laquelle les symboles sont définis comme à la revendication 1, suivie d'une sulfatation lorsqu'on veut obtenir un produit de formule générale (II) selon la revendication 1, dans laquelle R représente un radical $SO_3H$, puis isole le produit obtenu, soit à l'état libre soit à l'état de sel et le transforme, si on le désire, en un produit se présentant à l'état de sel ou à l'état libre.

## Patentansprüche

### Patentansprüche für die Vertragsstaaten : AT BE CH DE FR GB IT LI LU NL SE :

1. Neue Peptidderivate vom CCK8, dadurch gekennzeichnet, daß sie der allgemeinen Formel :

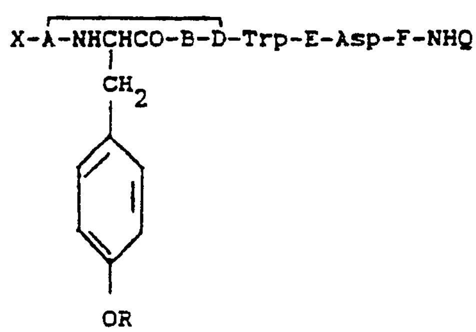

(II)

entsprechen, in welcher X ein Wasserstoffatom oder eine Schutzgruppe für die Aminfunktion vom Acyltyp oder Urethantyp darstellt, R ein Wasserstoffatom oder einen Rest —$SO_3H$ darstellt,

A und D die folgende Bedeutung haben :

a) A und D sind entweder verschieden und stellen einen Rest der allgemeinen Formel :

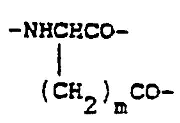 (III) oder 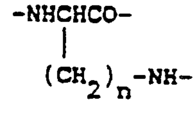 (IV)

dar, in welchen m und n ganze Zahlen von 1 bis 5 bedeuten, wobei D selbstverständlich einen Rest der allgemeinen Formel (IV) darstellt, wenn A einen Rest der allgemeinen Formel (III) darstellt und umgekehrt, und A und D zusammen mit dem Rest des Moleküls einen Ring durch Amidierung ihrer Seitenketten bilden, b) oder A und D sind gleich oder verschieden und stellen einen Rest der allgemeinen Formel :

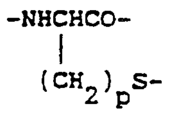 (V) oder 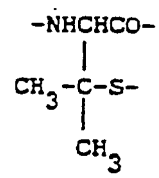 (VI)

dar, in welchen p eine ganze Zahl gleich 1 oder 2 darstellt, wobei A und D selbstverständlich zusammen mit dem Rest des Moleküls einen Ring durch Bildung einer Disulfidbrücke zwischen den Schwefelatomen und ihren Seitenketten bilden,

B und E, die gleich oder voneinander verschieden sind, einen Methionin-, Norleucin-, Leucin-, Serin-, Threonin-, Allothreonin- oder Cystein- oder Homocysteinrest bilden, deren OH—oder SH-Funktionen frei oder

i) durch einen Alkylrest mit 1 bis 6 kohlenstoffatomen in gerader oder verzweigter Kette oder
ii) durch einen gegebenenfalls durch ein oder mehrere Fluoratome substituierten Phenylrest oder
iii) durch einen gegebenenfalls durch ein oder mehrere Fluoratome substituierten Benzylrest oder
iv) durch einen Alkylcarbonylrest, dessen Alkylteil 1 bis 5 Kohlenstoffatome in gerader oder verzweigter Kette enthält, oder einen Benzoyl-, Phenylacetyl- oder Benzhydrylcarbonylrest, deren Phenylteile gegebenenenfalls durch ein oder mehrere Fluoratome substituiert sein können,

geschützt sein können,

F eine Valenzbindung oder einen Phenylalanin-, O-methylierten Tyrosin-, oder Cyclohexylalaninrest darstellt und

Q ein Wasserstoffatom oder einen Alkylrest mit 1 bis 6 Kohlenstoffatomen in gerader oder verzweigter Kette, gegebenenfalls substituiert durch ein oder mehrere Fluoratome, oder einen Phenyl- oder Phenylalkylrest, dessen Alkylteil 1 bis 3 Kohlenstoffatome in gerader oder verzweigter Kette enthält und dessen Phenylteil gegebenenfalls durch ein oder mehrere Fluoratome substituiert sein kann, darstellt, wobei die verschiedenen in die Definition der erfindungsgemäßen Produkte fallenden Aminosäuren in D-, L- oder DLForm vorliegen, sowie deren pharmazeutisch zulässige Salze.

2. Verfahren zur Herstellung der Peptide nach Anspruch 1, dadurch gekennzeichnet, daß man eine Säure der allgemeinen Formel

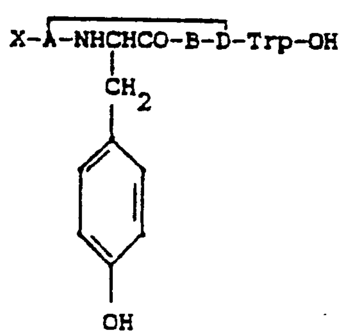

(VII)

in welcher die Symbole die im Anspruch 1 angegebene Bedeutung haben, mit einem Amin der allgemeinen Formel :

H-E-Asp-F-NHQ (VIII)

in welcher die Symbole die im Anspruch 1 angegebene Bedeutung haben, umsetzt, gefolgt von einer Sulfatierung, wenn man eine Verbindung der allgemeinen Formel (II) nach Anspruch 1 erhalten will, worin R einen Rest $SO_3H$ darstellt, und dann das erhaltene Produkt entweder im freien Zustand oder im Zustand des Salzes isoliert und es gewünschtenfalls in ein Produkt umwandelt,das im Zustand des Salzes oder im freien Zustand vorliegt.

3. Pharmazeutisches Präparat, dadurch gekennzeichnet, daß es zumindest eine Verbindung der allgemeinen Formel (II) der im Anspruch 1 angegebenen Bedeutung zusammen mit einem oder mehreren verträglichen und pharmazeutisch zulässigen Verdünnungsmitteln oder Zusatzstoffen enthält.

**Patentanspruch für die Vertragsstaaten ES GR :**

1. Verfahren zur Herstellung von neuen Peptidderivaten von CCK8 der allgemeinen Formel :

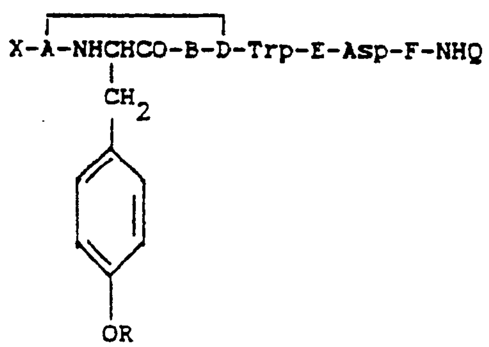

(II)

in welcher X ein Wasserstoffatom oder eine Schutzgruppe für die Aminfunktion vom Acyltyp oder Urethantyp derstellt, R ein Wasserstoffatom oder einen Rest—$SO_3H$ darstellt,

A und D die folgende Bedeutung haben :

a) A und D sind entweder verschieden und sstellen einen Rest der allgemeinen Formel :

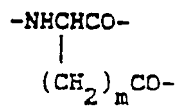 (III) oder 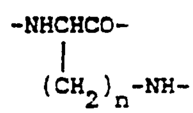 (IV)

dar, in welchen m und n ganze Zahlen von 1 bis 5 bedeuten, wobei D selbstverständlich einen Rest der allgemeinen Formel (IV) darstellt, wenn A einen Rest der allgemeinen Formel (III) darstellt und umgekehrt, und A und D zusammen mit dem Rest des Moleküls einen Ring durch Amidierung ihrer Seitenketten bilden,

b) oder A und D sind gleich oder verschieden und stellen einen Rest der allgemeinen Formel :

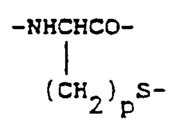 (V) oder 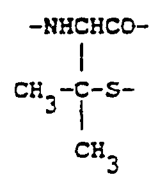 (VI)

dar, in welchen p eine ganze Zahl gleich 1 oder 2 darstellt, wobei A und D selbstverständlich zusammen mit dem Rest des Moleküls eine Ring durch Bildung einer Disulfidbrücke zwischen den Schwefelatomen und ihren Seitenketten bilden,

B und E, die gleich oder voneinander verschieden sind, einen Methionin-, Norleucin-, Leucin-, Serin-, Threonin-, Allothreonin-, oder Cystein- oder Homocysteinrest bilden, deren OH— oder SH-Funktionen frei oder

i) durch einen Alkylrest mit 1 bis 6 kohlenstoffatomen in gerader oder verzweigter Kette oder

ii) durch einen gegebenenfalls durch ein oder mehrere Fluoratome substituierten Phenylrest oder

iii) durch einen gegebenenfalls durch ein oder mehrere Fluoratome substituierten Benzulrest oder

iv) durch einen Alkylcarbonylrest, dessen Alkylteil 1 bis 5 Kohlenstoffatome in gerader oder verzweigter Kette enthält, oder einen Benzoyl-, Phenylacetyl- oder Benzhydrylcarbonylrest, deren Phenylteile gegebenenenfalls durch ein oder mehrere Fluoratome substituiert sein können,

geschützt sein können,

F eine Valenzbindung oder einen Phenylalanin-, O-methylierten Tyrosin-, oder Cyclogexylalaninrest darstellt und

Q ein Wasserstoffatom oder einen Alkylrest mit 1 bis 6 Kohlenstoffatomen in gerader oder verzweigter Kette, gegebenenfalls substituiert durch ein oder mehrere Fluoratome, oder einen Phenyl- oder Phenylalkylrest, dessen Alkylteil 1 bis 3 Kohlenstoffatome in gerader oder verzweigter Kette enthält und dessen Phenylteil gegebenenfalls durch ein oder mehrere Fluoratome substituiert sein kann, darstellt, wobei die verschiedenen in die Definition der erfindungsgemäßen Produkte fallender Aminosäuren in D—, L— oder DL—Form vorliegen, sowie von deren pharmazeutisch zulässigen Salzen, dadurch gekennzeichnet, daß man eine Säure der allgemeinen Formel

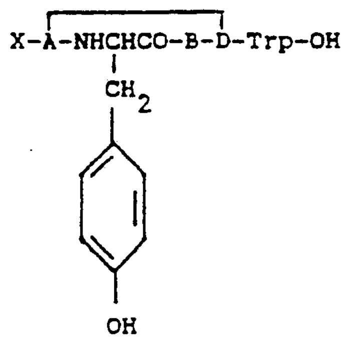

(VII)

in welcher die Symbole die im Anspruch 1 angegebene Bedeutung haben, mit einem Amin der allgemeinen Formel :

H-E-Asp-F-NHQ (VIII)

in welcher die Symbole die im Anspruch 1 angegebene Bedeutung haben, umsetzt, gefolgt von einer Sulfatierun, wenn man eine Verbindung der allgemeinen Formel (II) nach Anspruch 1 erhalten will, worin R einen Rest $SO_3H$ darstellt, und dann das erhaltene Produkt entweder im freien Zustand oder im Zustand des Salzes isoliert und es gewünschtenfalls in ein Produkt umwandelt, das im Zustand des Salzes oder im freien Zustand vorliegt.

## Claims

### Claims for the contracting states : AT BE CH DE FR GB IT LI LU NL SE

1. New peptides derived from CCK8, characterized in that they correspond to the general formula :

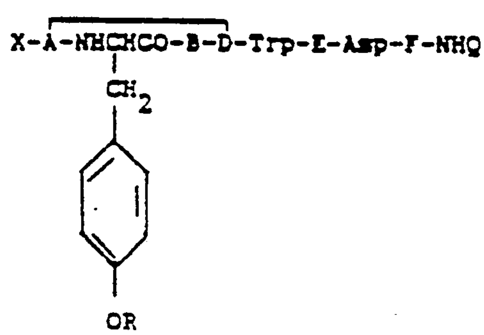

(II)

in which X denotes a hydrogen atom or alternatively a radical which protects the amine group of the acyl type, or of the urethane type,

R denotes a hydrogen atom or an —$SO_3H$ radical,

A and D are defined as follows :

a) either A and D are different and denote a radical of general formula :

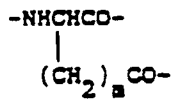 (III) or 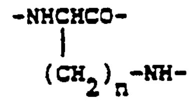 (IV)

in which m and n denote integers between 1 and 5, on the understanding that, when A denotes a radical of general formula (III), D denotes a radical of general formula (IV), and vice versa, and that A and D form, together with the remainder of the molecule, a ring by amidation of their side chains,

b) or A and D are identical or different and denote a radical of general formula :

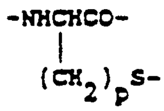 (V) or 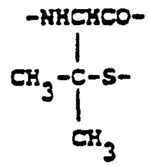 (VI)

in which p denotes an integer equal to 1 or 2, on the understanding that A and D form, together with the remainder of the molecule, a ring by formation of a disulphide bridge between the sulphur atoms and their side chains,

B and E, which may be identical or different, denote a methionine, norleucine, leucine, serine, threonine or allo-threonine residue, or alternatively a cysteine or homocysteine residue in which the OH or SH groups can be free or protected

i) by a straight- or branched-chain alkyl radical containing 1 to 6 carbon atoms or

ii) by a phenyl radical, unsubstituted or substituted with one or more fluorine atoms, or

iii) by a benzyl radical, unsubstituted or substituted with one or more fluorine atoms, or

iv) by an alkylcarbonyl radical in which the alkyl portion contains 1 to 5 carbon atoms in a straight or branched chain, or alternatively a benzoyl, phenylacetyl or benzhydrylcarbonyl radical in which the phenyl portions can be unsubstituted or substituted with one or more fluorine atoms,

F denotes a valency bond or a phenylalanine, O-methyltyrosine or cyclohexylalanine residue,

and Q denotes a hydrogen atom or an alkyl radical containing 1 to 6 carbon atoms in a straight or branched chain, unsubstituted or substituted with one or more fluorine atoms, or a phenyl or phenylalkyl radical in which the alkyl portion contains 1 to 3 carbon atoms in a straight or branched chain and in which the phenyl portion can be unsubstituted or substituted with one or more fluorine atoms, on the understanding that the different amino acids falling within the definition of the products according to the invention are in D, L or DL form, as well as their pharmaceutically acceptable salts.

2. Process for preparing peptides according to Claim 1, characterized in that an acid of general formula :

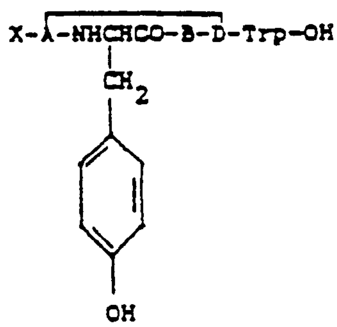 (VII)

in which the symbols are defined as in Claim 1, is reacted with an amine of general formula :

H-E-Asp-F-NHQ (VIII)

in which the symbols are defined as in Claim 1, followed by a sulphation when it is desired to obtain a product of general formula (II) according to Claim 1 in which R denotes an $SO_3H$ radical, and the product obtained is then isolated either in the free state or in the state of a salt and converted, if so desired, to a product which is in the state of a salt or in the free state.

3. Pharmaceutical composition, characterized in that it contains at least one product of general formula (II)

defined as in Claim 1, in combination with one or more diluents or adjuvants that are compatible and pharmaceutically acceptable.

**Claims for the contracting states : ES GR**

1. Process for preparing new peptides derived from CCK8, of general formula :

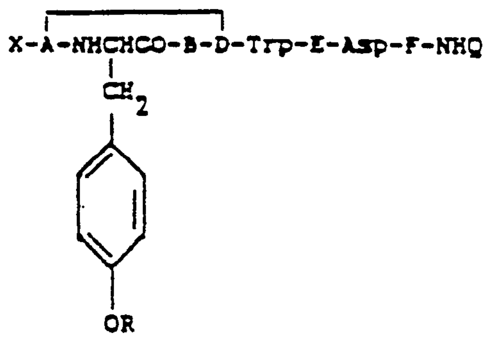

(II)

in which X denotes a hydrogen atom or alternatively a radical which protects the amine group of the acyl type, or of the urethane type,
R denotes a hydrogen atom or an —$SO_3H$ radical,
A and D are defined as follows :
a) either A and D are different and denote a radical of general formula :

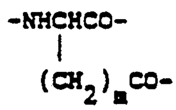

(III) or

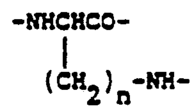

(IV)

in which m and n denote integers between 1 and 5, on the understanding that, when A denotes a radical of general formula (III), D denotes a radical of general formula (IV), and vice versa, and that A and D form, together with the remainder of the molecule, a ring by amidation of their side chains,
b) or A and D are identical or different and denote a radical of general formula :

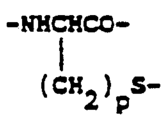

(V) or

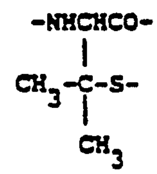

(VI)

in which p denotes an integer equal to 1 or 2, on the understanding that A and D form, together with the remainder of the molecule, a ring by formation of a disulphide bridge between the sulphur atoms and their side chains,
B and E, which may be identical or different, denote a methionine, norleucine, leucine, serine, threonine or allo-threonine residue, or alternatively a cysteine or homocysteine residue in which the OH or SH groups can be free or protected

i) by a straight- or branched-chain alkyl radical containing 1 to 6 carbon atoms or
ii) by a phenyl radical, unsubstituted or substituted with one or more fluorine atoms, or
iii) by a benzyl radical, unsubstituted or substituted with one or more fluorine atoms, or
iv) by an alkylcarbonyl radical in which the alkyl portion contains 1 to 5 carbon atoms in a straight or branched chain, or alternatively a benzoyl, phenylacetyl or benzhydrylcarbonyl radical in which the phenyl portions can be unsubstituted or substituted with one or more fluorine atoms,

F denotes a valency bond or a phenylalanine, O-methyltyrosine or cyclohexylalanine residue,
and Q denotes a hydrogen atom or an alkyl radical containing 1 to 6 carbon atoms in a straight or branched chain, unsubstituted or substituted with one or more fluorine atoms, or a phenyl or phenylalkyl radical in which the alkyl portion contains 1 to 3 carbon atoms in a straight or branched chain and in which the phenyl

portion can be unsubstituted or substituted with one or more fluorine atoms, on the understanding that the different amino acids falling within the definition of the products according to the invention are in D, L or DL form, as well as their pharmaceutically acceptable salts, characterized in that an acid of general formula:

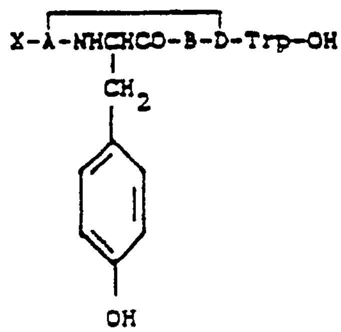

(VII)

in which the symbols are defined as in Claim 1 (sic), is reacted with an amine of general formula :

H-E-Asp-F-NHQ (VIII)

in which the symbols are defined as in Claim 1 (sic), followed by a sulphation when it is desired to obtain a product of general formula (II) according to Claim 1 (sic) in which R denotes an $SO_3H$ radical, and the product obtained is then isolated either in the free state or in the state of a salt and converted, if so desired, to a product which is in the state of a salt or in the free state.